# EUROPÄISCHE PATENTSCHRIFT

(11) **EP 0 744 944 B1**
(45) Veröffentlichungstag und Bekanntmachung des Hinweises auf die Patenterteilung: **03.11.1999**
(21) Anmeldenummer: 95908925.1
(22) Anmeldetag: 09.02.1995
(51) Int. Cl.: A61K 9/70, A61K 47/26, A61K 31/565, A61K 31/57

(54) **TRANSDERMALE THERAPEUTISCHE SYSTEME ENTHALTEND SEXUALSTEROIDE**
SEXUAL STEROID-CONTAINING TRANSDERMAL THERAPEUTIC SYSTEMS
SYSTEMES THERAPEUTIQUES TRANSDERMIQUES CONTENANT DES STEROIDES SEXUELS

(30) Priorität: 18.02.1994 DE 4405898
(43) Veröffentlichungstag der Anmeldung: 04.12.1996
(73) Patentinhaber: SCHERING AKTIENGESELLSCHAFT, 13342 Berlin (DE)
(72) Erfinder: LIPP, Ralph, D-10717 Berlin (DE); GÜNTHER, Clemens, D-13357 Berlin (DE); RIEDL, Jutta, D-10557 Berlin (DE); TÄUBER, Ulrich, D-13359 Berlin (DE)
(86) Internationale Anmeldenummer: EP9500483
(87) Internationale Veröffentlichungsnummer: WO9522322

(56) Entgegenhaltungen:
- EP-A- 0 137 278
- EP-A- 0 288 734
- EP-A- 0 421 454
- EP-A- 0 461 290
- WO-A-89/04179
- US-A- 4 082 881

## Beschreibung

Die Erfindung betrifft transdermale therapeutische Systeme enthaltend Sexualsteroide und gegebenenfalls Penetrationsverstärker und Kristallisations-inhibitoren, welche dadurch gekennzeichnet sind, daß sie Dimethylisosorbid enthalten mit Ausnahme von Systemen, die wirkstoffhaltige nicht fließfähige Gelphasen oder 3-Ketodesogestrel enthalten.
Erfindungsgemäß sollen unter Sexualsteroiden vorzugsweise Gestagene und/oder Östrogene verstanden werden, obgleich grundsätzlich auch andere Sexualsteroide, wie Androgene, Antiöstrogene oder Antigestagene zur Herstellung der erfindungsgemäßen Mittel geeignet sind.

Geeignete Gestagene für das erfindungsgmäße Mittel sind beispielsweise das Gestoden, das Levonorgestrel, das Desogestrel, das Norethisteron und das Norethisteronacetat. Zur Herstellung der erfindungsgemäßen Mittel eignet sich auch das 3-Keto-desogestrel, wie man aus der zum Prioritätszeitpunkt der vorliegenden Anmeldung noch nicht vorpublizierten PCT/EP93/02224 entnehmen kann.

Geeignete Östrogene für das erfindungsgemäße Mittel sind beispielsweise das Estradiol, das Estriol, das Ethinylestradiol, das Mestranol, das 14α,17α-Ethanoestra-1,3,5(10)-trien 3,17β-diol (WO 88/01275), das 14α,17α-Ethanoestra-1,3,5(10)-trien-3β,16α,17α-ethanoestra-1,3,5(10)-trien-3β,16α,17α-triol (WO91/08219) und deren Ester (EP-A 163596), wie das Estradiol-dipropionat, das Estradiol-dihexanoat und das Estradiol-didecanoat. Die erfindungsgemäßen Kombinationspräparate enthalten neben mindestens einem Gestagen vorzugsweise 1 bis 3 - insbesondere 1 bis 2 Östrogen(e).

Die EP-B 0 137 278, die transdermale therapeutische Systeme betrifft, bei denen der Wirkstoff in einer nicht fließfähigen Gelphase eingebettet ist, enthält einen allgemein gehaltenen Hinweis, daß man in ihren als Lösungsmittel auch Dimethylisosorbid verwenden könne, ohne daß aus der Schritt entnommen werden könnte, daß die Vewendung dieses Mittels bei transdermalen therapeutischen Systemen von besonderem Nutzen sein könnte.

Transdermal zu applizierende therapeutische Systeme haben bekanntlich den Vorzug, daß sie über einen längeren Zeitraum hin eine gleichmäßigere Freisetzung des Wirkstoffs ermöglichen, als dies in der Regel bei anders - wie zum Beispiel peroral - zu applizierenden Mitteln möglich ist. Diese Eigenschaften lassen sich in einer Reihe von endokrinen Erkrankungen vorteilhaft ausnutzen. Für in Wasser schwer lösliche Steroidhormone, wie zum Beispiel die Gestagene, ist es aber in der Regel recht problematisch, transdermale Systeme zu erstellen, die eine zur Therapie ausreichende Penetration des Wirkstoffs durch die Haut gewährleisten.

Es wurde nun gefunden, daß es mit Hilfe des erfindungsgemäßen Mittels, welches neben dem/n Sexualsteroid(en) zusätzlich Dimethylisosorbid enthält, überraschenderweise möglich ist, eine therapeutisch ausreichende sehr gleichmäßige Penetrationsgeschwindigkeit der Steroidhormone durch die Haut zu erzielen, während dies bei den bekannten Steroidhormone enthaltenden transdermal zu applizierenden Mitteln nur bedingt möglich ist. (EP-A 137278 und EP-A 275716), was den Einsatz von vergleichsweise großen Systemen notwendig macht.

Dimethylisosorbid ist eine Substanz der Formel

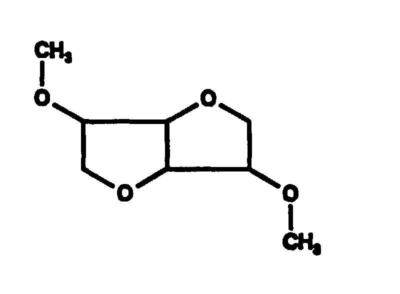

Es ist bekanntlich eine Substanz mit gutem Lösungsvermögen für organische Verbindungen (H. P. Fiedler, Lexikon der Hilfsstoffe für Pharmazie, Kosmetik und angrenzende Gebiete, Editio Cantor Aulendorf, 1989). Dieses wird beispielsweise in USP 4082881 ausgenutzt, um in verschiedenen topischen Zubereitungen, nicht jedoch in Transdermalsystemen, hohe Konzentrationen organischer Arzneistoffe gelöst zu halten. In USP 4814173 wird Dimethylisosorbid als Solvens für verschiedene Arzneistoffe, nicht jedoch für Sexualsteroide, in Lösungen, Emulsionen sowie in kompliziert aufgebauten Transdermalen Therapeutischen Systemen auf der Basis von Silikonhauthaftklebstoffen eingesetzt.

Es wurde nun festgesteltt, daß Dimethylisosorbid eine Reihe von Sexualsteroiden in beachtlichem Umfang zu lösen vermag. Eine Auswahl dieser Steroide ist in Tab. 1 wiedergegeben. Insbesondere das Levonorgestrel, welches in den meisten Standardvehikeln zur transdermalen Anwendung nur gering löslich ist, kann in Dimethylisosorbid in deutlich höheren Konzentrationen gelöst werden.

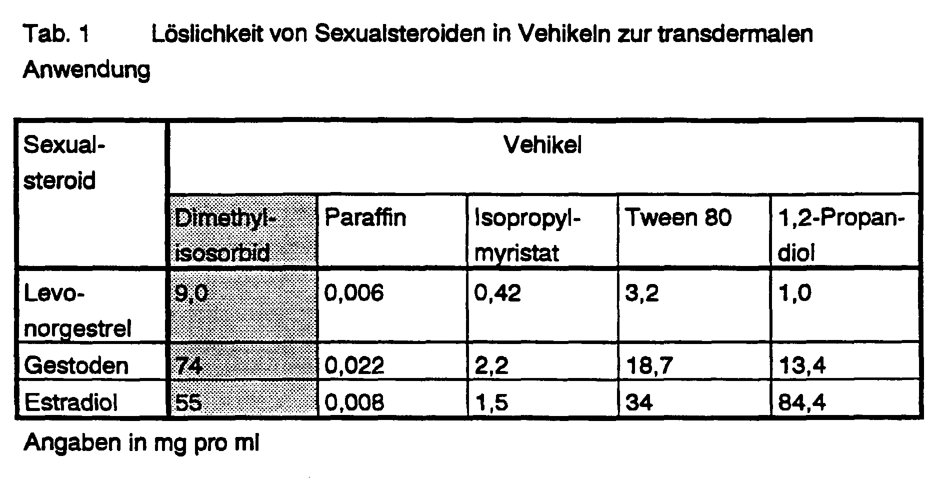

Darüber hinaus nivelliert Dimethylisosorbid die Löslichkeiten der Gestagene und Östrogene. Dies ist besonders für die Entwicklung von Kombinationstransdermalsystemen ein großer zusätzlicher Vorteil, da hierdurch erstmals hochkonzentrierte binäre Gemische von Gestagenen und Östrogenen in vorteilhaften Beladungsverhältnissen von ca. 5:1 bis 1:5 gemeinsam in einer Matrix molekulardispers verteilt werden können. Hierdurch wird sichergestellt, daß beide Wirkstoffe gleichzeitig in hoher thermodynamischer Aktivität im System vorliegen.

Dimethylisosorbid ist, wie in DD-A- 217989 beschrieben, in geeignetem Umfang mit zur Herstellung von Transdermalsystemen üblichen Hauthaftklebem mischbar.

Es wurde nun gefunden, daß bestimmte Mischsysteme aus Hauthaftkleber und Dimethylisosorbid im Vergleich zu Systemen ohne Dimethylisosorbid ein deutlich gesteigertes Lösungsvermögen für Sexualsteroide aufweisen. Diese neuen Systeme, deren Aufbau in Fig. 1 dargestellt ist, sind im Gegensatz zu den in der EP-A 0 137 278 genannten Sexualsteroid-haltigen Silopren-Systemen einphasig und zeichnen sich dadurch aus, daß sie selbständig auf der Haut kleben. Auch von den in WO 89/04179 genannten topischen Zubereitungen, insbesondere den dort genannten wirkstoffhaltigen Pflastern, heben sie sich aufgrund ihres einfachen Aufbaus ab. Sie sind einfacher herzustellen als die vorgenannten Transdermalsysteme und zeichnen sich durch günstigere Trageeigenschaften bedingt durch ihre dünne, flexible Konstruktion aus.

Neben seinen guten Lösungseigenschaften für organische Moleküle, besitzt Dimethylisosorbid auch penetrationsverstärkende Eigenschaften. So haben in vitro-Penetrationsuntersuchungen überraschenderweise ergeben, daß das Vehikel Dimethylisosorbid starke penetrationsfördernde Eigenschaften für Sexualsteroide besitzt (s. Tab. 2).

In den erfindungsgemäßen Mitteln werden normalerweise 1 bis 40 Gewichtsprozent und vorzugsweise 5 bis 25 Gewichtsprozent Dimethylisosorbid, bezogen auf die gesamte Wirkstoffphase, verwendet.

**Tab. 2**

| Perkutane Resorption (in µg/cm²/h) von Sexualsteroiden in Formulierungen ohne Penetrationsverstärker durch Haut der haarlosen Maus | | | | |
|---|---|---|---|---|
| | | **Mittlerer Fluß** | | **Maximaler Fluß** |
| **Steroid** | **Formulierung** | **Tag 1** | **Tag 2** | |
| 0.2 mg LN | PG | 0.18 ± 0.06 | 0.53 ± 0.07 | 0.74 ± 0.14 |
| 0.2 mg LN | PGML | 0.25 ± 0.06 | 0.42 ± 0.03 | 0.64 ± 0.06 |
| 0.2 mg LN | DMI | 0.31 ± 0.14 | 0.66 ± 0.10 | 1.23 ± 0.32 |
| 1 mg LN | DMI | 0.62 ± 0.20 | 1.02 ± 0.40 | 1.62 ± 0.61 |
| 0.2 mg LN | IPM | 0.13 | 0.11 | 0.16 |
| 0.2 mg E₂ | DMI | 0.51 ± 0.17 | 0.79 ± 0.48 | 0.97 ± 0.57 |
| 1 mg E₂ | DMI | 2.63 ± 1.21 | 3.10 ± 1.99 | 4.17 ± 1.57 |
| 0.2 mg E₂ | PG | 0.92 ± 0.26 | 0.25 ± 0.01 | 1.34 ± 0.24 |
| 0.2 mg E₂ | IPM | 0.47 | 0.29 | 0.69 |
| DMI: Dimethylisosorbid IPM: Isopropylmyristat PG: Propylenglykol PGML: Propylenglykolmonolaurat LN: Levonorgestrel E2: Estradiol | | | | |

Durch geeignete Kombination von Dimethylisosorbid mit anderen, bekannten Penetrationsverstärkern lassen sich die in vitro erzielten transdermalen Flüsse der Sexualsteroide sogar noch weiter steigern (s. Tab. 3).

**Tab. 3**

| Perkutane Resorption (in µg/cm²/h) von Sexualsteroiden in Formulierungen mit Penetrationsverstärkern durch Haut der haarlosen Maus | | | | |
|---|---|---|---|---|
| | | **Mittlerer Fluß** | | **Maximaler Fluß** |
| **Steroid** | **Formulierung** | **Tag 1** | **Tag 2** | |
| 0.2 mg LN | DMI+10%LA | 1.70 ± 0.18 | 0.91 ± 0.16 | 3.62 ± 0.76 |
| 0.2 mg LN | DMI+10%LS | 1.95 ± 0.81 | 1.27 ± 0.62 | 3.28 ± 1.40 |
| 0.2 mg LN | PG+10%LA | 0.67 ± 0.04 | 0.64 ± 0.11 | 1.32 ± 0.05 |
| 0.2 mg LN | PG+5%Azone | 0.71 ± 0.22 | 0.75 ± 0.16 | 1.13 ± 0.43 |
| 0.2 mg E2 | DMI+10%LA | 7.35 ± 1.15 | 4.86 ± 1.97 | 11.57 ± 1.72 |
| 0.2 mg E2 | DMI+10%LS | 9.06 ± 0.50 | 7.55 ± 0.33 | 15.29 ± 0.61 |
| 0.2 mg E2 | PG+5%Azone | 1.92 ± 0.18 | 0.90 ± 0.10 | 5.15 ± 0.84 |
| LA: Laurylalkohol LS: Laurinsäure | | | | |

Mit Hilfe des erfindungsgemäßen Mittels können Transdermale Therapeutische Systeme mit hohen Konzentrationen an molekulardispers gelösten Sexualsteroiden hergestellt werden. Bei der Anwendung dieser neuen Systeme wird ein besonders hoher wirksamer Konzentrationsgradient zwischen Arzneimittel und Haut erreicht. Der hohe Konzentrationsgradient und die starke penetrationsfördernde Wirkung des Dimethylisosorbids respektive der Dimethylisosorbid-Enhancer-Kombinationen bewirken gemeinsam die hohen transdermalen Flüsse der verarbeiteten Steroidhormone.

Im Einzelfall kann ein Zusatz von Kristallisationsinhibitoren zur Matrix der vorgenannten Systeme, wie in der WO 93/08795 beschrieben, die Lagerstabilität der erfindungsgemäßen Transdermalsysteme verbessern.

Eine sehr gleichmäßige Applikation mit eingestellter Dosierung des Wirkstoffes oder der Wirkstoffgemische kann man erzielen, wenn man den Wirkstoff oder das Gemisch in ein transdermales therapeutisches System (TTS) und hier speziell in ein Matrixsystem einbettet. Geeignete Matrixsysteme sind solche, die man üblicherweise zur percutanen Applikation von Wirkstoffen anwendet (Yie W. Chien: "Transdermal Controlled Systemic Medications", Marcel Dekker, Inc., New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Research 1030 Hamilton Court Menlo Park CA 94025 (415) 328-2228).

So kann man beispielsweise ein solches transdermales therapeutisches System verwenden, welches aus
a) einer undurchlässigen Deckschicht,
   ein bis drei an der Deckschicht haftenden, das Gestagen und/oder das Östrogen, und das Dimethylisosorbid und gewünschtenfalls penetrationsverstärkende Mittel und einen oder mehrere Kristallisationsinhibitoren enthaltende, für diese Komponenten durchlässigen selbsthaftenden oder von einem gewünschtenfalls penetrationsverstärkende Mittel enthaltenden Hauthaftkleber abgedecktem oder umgebenen Matrixschicht(en), einer abziehbaren Schutzschicht, oder
b) einer mit einem gewünschtenfalls penetrationsverstärkende Mittel enthaltenden Haftkleber versehen Abdeckung,
   ein oder zwei eine Haftkleberumrandung unbedeckt lassende, mittels einer Abdeckung an dem Haftkleber befestigten, das Gestagen und/oder das Östrogen, und das Dimethylisosorbid und gegebenenfalls penetrationsverstärkende Mittel und Kristallisationsinhibitoren enthaltende Matrixschicht(en) und einer abziehbaren Schutzschicht, besteht.

Ein transdermales therapeutisches System gemäß Variante a) stellt ein einfaches Matrixsystem dar. Es kann beispielsweise von runder, ovaler oder rechteckiger Form sein und wie folgt hergestellt werden.

Eine Lösung von bis zu 25 Gewichtsprozent Wirkstoff oder Wirkstoffgemisch, 1-40 Gewichtsprozent Dimethylisosorbid oder eines Gemisches aus Dimethylisosorbid und anderen penetrationsverstärkenden Mitteln, 30-70 Gewichtsprozent eines medizinisch üblichen Klebers aufgefüllt mit einem geeigneten flüchtigen Lösungsmittels zu 100 Gewichtsprozent wird auf eine plane undurchlässige Deckschicht gestrichen. Nach dem Trocknen kann auf diese Schicht eine zweite und gewünschtenfalls später sogar eine dritte gegebenenfalls Wirkstoff, penetrationsverstärkende Mittel und Kleber enthaltende Schicht aufgetragen und getrocknet werden. Dann wird das Matrixsystem mit einer abziehbaren Schutzschicht versehen.

Verwendet man einen medizinisch üblichen Matrixbildner, der nach dem Trocknen des Systems nicht oder nicht ausreichend auf der Haut haftet, so kann man das System vor dem Aufbringen der abziehbaren Schutzschicht noch zusätzlich mit einem Hauthaftkleber abdecken oder umgeben.

Geeignete flüchtige Lösungsmittel sind beispielsweise niedere Alkohole, Ketone oder niedere Carbonsäureester wie Ethanol, Isopropanol, Aceton oder Ethylacetat, polare Ether, wie Tetrahydrofuran, niedere Kohlenwasserstoffe, wie Cyclohexan oder Benzin oder auch Halogenkohlenwasserstoffe, wie Dichlormethan, Trichlormethan, Trichlortrifluorethan und Trichlorfluormethan. Es bedarf keiner Erläuterung, daß auch Gemische dieser Lösungsmittel geeignet sind.

Geeignete penetrationsverstärkende Mittel sind beispielsweise ein- oder mehrwertige Alkohole, wie Ethanol, 1,2-Propandiol oder Benzylalkohol, gesättigte und ungesättigte Fettalkohole mit 8 bis 18 Kohlenstoffatomen, wie Laurylalkohol oder Cetylalkohol, Kohlenwasserstoffe, wie Mineralöl, gesättigte und ungesättigte Fettsäuren mit 8 bis 18 Kohlenstoffatomen, wie Stearinsäure oder Ölsäure, Fettsäureester mit bis zu 24 Kohlenstoffatomen oder Dicarbonsäurediester mit bis zu 24 Kohlenstoffatomen.

Als medizinisch übliche Kleber eigenen sich beispielsweise Polyacrylate, Silicone, Polyurethane, Blockpolymere, Styrol-Butadien-Kopolymere sowie natürliche oder synthetische Kautschuke, wie z.B. Polyisobutylene und insbesondere Polyacrylate. Als weitere Matrixbildner kommen Celluloseether, Polyvinylverbindungen oder Silikate in Betracht. Zur Erhöhung der Klebrigkeit können der erhaltenen Matrix die üblichen Additive, wie zum Beispiel klebrig machende Harze und Öle zugesetzt werden.

Fettsäureester, die sich für das erfindungsgemäße Mittel eignen, sind beispielsweise solche der Essigsäure, Capronsäure, Laurinsäure, Myristinsäure, Stearinsäure und Palmitinsäure, wie zum Beispiel die Methylester, Ethylester, Propylester, Isopropylester, Butylester, sec.-Butylester, Isobutylester, tert.-Butylester oder Monoglycerinsäureester dieser Säuren. Besonders bevorzugte Ester sind solche der Myristinsäure, oder Ölsäure wie deren Methylester und insbesondere deren Isopropylester. Geeignete Dicarbonsäurediester sind beispielsweise das Diisopropyladipat, Diisobutyladipat und Diisopropylsebacat.

Weitere penetrationsverstärkende Mittel sind Phosphatidderivate, wie das Lecitin, Terpene, Amide, Ketone, Harnstoff und seine Derivate oder Ether wie zum Beispiel Dimethylisosorbid und Diethylenglycolmonoethylether. Es bedarf keiner näheren Erläuterung, das auch Gemische dieser penetrationsverstär-kenden Mittel zur Herstellung des erfindungsgemäßen Mittels geeignet sind.

Als Kristallisationsinhibitoren, die die Lagerstabilität der erfindungsgemäßen Systeme im Einzelfall verbessern können, eignen sich beispielsweise Zusätze von hochdispersem Siliciumdioxid oder makromolekularen Stoffen wie Polyvinylpyrrolidon (beispielsweise Kollidon 12 PF, Kollidon 17 PF, Kollidon 25 und Kollidon 30 der BASF), Vinylpyrrolidon-Vinylacetat-Copolymer (beispielsweise Kollidon VA 54 der BASF), quervernetztes Poylvinylpyrrolidon, Polyvinylalkohol, Hydroxypropylcellulose, Ethylcellulose, Gelatine, Stärke(derivate) und Dextran.

Als Schutzschicht eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielswiese 10 bis 100 µm dicke Folien aus Polyethylen oder Polyester wahlweise pigmentiert oder metallisiert verwenden. Die hierauf aufgebrachte Arzneimittelschicht hat vorzugsweise eine Dicke von 20 bis 500 µm. Die Abgabe der Wirkstoffe erfolgt üblicherweise über eine Fläche von 1-100 cm² und vorzugsweise von unter 5 bis 100 cm².

Bei mehrschichtigen Matrixsystemen kann beispielsweise in die an der undurchlässigen Deckschicht aufgetragenen Matrix das Gestagen, das Dimethylisosorbid und gegebenenfalls die Penetrationsverstärker eingebracht werden, während die darunter befindliche Schicht oder Schichten das Östrogen nebst Dimethylisosorbid und gegebenenfalls ebenfalls Penetrationsverstärker enthält. Andererseits ist es aber auch möglich, in einem solchen transdermalen System mehrere wirkstoffhaltige Matrixsysteme nebeneinander anzuordnen.

Ein transdermales therapeutisches Matrixsystem gemäß Variante b kann beispielsweise ebenfalls rund, oval oder rechteckig sein und wie folgt hergestellt werden:

Eine Abdeckung wird mit einem Hauthaftkleber beschichtet. Dann klebt man auf diese pro TTS ein oder zwei ausgestanzte Areale einer mit einer undurchlässigen Abdeckung versehenen, das Gestagen, das Dimethylisosorbid, gegebenenfalls das oder die Östrogen(e) und ggf. penetrationsverstärkende Mittel enthaltende Matrixschichten so auf, daß die Abdeckung einen ausreichenden Rand zur Befestigung auf der Haut und bei mehreren Arealen auch ausreichende Zwischenräume besitzt und versieht sie mit einer abziehbaren Schutzschicht. Die in diesen Matrixsystem verwendeten Materialien können die gleichen sein, wie in denjenigen der Variante a.

Es können bei diesem System neben Dimethylisosorbid die oben erwähnten penetrationsverstärkenden Mittel angewendet werden. Als durchlässige Polymerschicht wird beispielsweise eine 20 bis 200µm dicke Folie aus Celluloseestern, Celluloseethern, Siliconen oder Polyolefinverbindungen verwendet. Durch Variation dieser Polymerschicht läßt sich die Diffusionsgeschwindigkeit des Wirkstoffes oder Wirkstoffgemisches innerhalb weiter Grenzen variieren.

Als Kleber und Schutzschicht eignen sich die gleichen Materialien, die bei dem transdermalen therapeutischen System gemäß Variante a beschrieben sind.

Bei der Herstellung von transdermalen therapeutischen Systemen mit zwei nebeneinander angeordneten wirkstoffhaltigen Matrixschichten oder Arzneimittelreservoiren ist es oft zweimäßig, in dem einen das Gestagen und in dem anderen das Östrogen einzubringen. In derartigen Fällen können die wirkstoffhaltigen Matrixsysteme oder Arzneimittelreservoire nicht nur unterschiedliche Wirkstoffe, sondern zusätzlich auch noch unterschiedliche penetrationsverstärkende Mittel enthalten.

Bei den Marixsystemen gemäß Variante a oder b muß man für einen ausreichenden Abstand der Areale Sorge tragen um eine Diffusion der Wirkstoffe in das jeweils andere Areal zu unterbinden.

Weitere Merkmale der erfindungsgemäßen transdermalen Systeme seien anhand der beigefügten, nicht maßstabgerechten Zeichnungen erläutert.
Fig. 1 zeigt einen Querschnitt durch ein einfaches rundes Matrixsystem gemäß Variante a ohne die abziehbare Schutzschicht. Es besteht aus der undurchlässigen Deckschicht 1 und der arzneimittelhaltigen Matrixschicht 2.
Fig. 2 zeigt einen Querschnitt durch ein Matrixsystem gemäß Variante b ohne die abziehbare Schutzschicht.
Fig. 3 zeigt einen Längsschnitt durch dieses System. Das System besteht aus der Abdeckung 3, die mit einer Haftkleberschicht 4 versehen ist. An dieser Haftkleberschicht sind mittels undurchlässiger Abdeckungen 5 und 7 zwei arzneimittelhaltige Matrixschichten 6 und 8 befestigt.

Die erfindungsgemäßen Gestagen-Mittel zur transdermalen Applikation können zur Behandlung der gleichen Erkrankungen angewendet werden, wie die vorbekannten, beispielsweise oral zu applizierenden Mittel die hochwirksame Gestagene enthalten. Darüber hinaus können die erfindungsgemäßen gegebenenfalls östrogenhaltigen Präparate auch zur Kontrazeption und zur Substitutionstherapie in der Postmenopause Anwendung finden. Besondere Vorteile haben die erfindungsgemäßen Mittel bei der Behandlung von Erkrankungen, die eine Langzeitbehandlung mit relativ hoher Dosierung der Wirkstoffe erfordern. Hier kann die Applikationsfrequenz wesentlich verringert werden und ein wesentlich gleichmäßiger Blutplasmaspielgel erzielt werden. Vorteilhaft ist ferner, daß gastrointestinale Nebenwirkungen nicht zu erwarten sind und bei östrogenhaltigen Kombinationspräparaten die erste Leberpassage umgangen wird und daß die Dosis an Östrogen vermindert werden kann.

Diese Vorteile lassen die gestagenhaltigen Monotherapeutika der vorliegenden Erfindung als besonders geeignet erscheinen um beispielsweise die Endometriose, gestagenabhängige Tumore, benigne Brusterkrankungen oder das prämenstruelle Syndrom zu behandeln.

Die transdermale Anwendung von Östrogenen gegebenenfalls in sequentieller oder kontinuierlicher Kombination mit Gestagen bietet besondere Vorteile, beispielsweise zur Behandlung klimakterischer Beschwerden, zur Präventation der Osteoporose, zur Zyklusregulierung und zur Zyklusstabilisation.

Die nachfolgenden Ausführungsbeispiele dienen zur näheren Erläuterung der Erfindung. In ihnen wurden folgende Handelsprodukte verwendet:

Polyesterfolie von 0,074 mm Dicke (Skotchpak ® 1009 des Herstellers 3M; Polypropylenfolie (Celgard ® 2500) des Herstellers Celanese, Linerfolie Skotchpak ® 1022 und 1360 vom Hersteller 3M; Transferkleber 9871 vom Hersteller 3M, Polyacrylester-Kleber vom Typ Sichello ® J 6610-21 des Herstellers Henkel KG, Silikonklebstoff vom Typ X-7-2960 des Herstellers Dow Corning und Hydroxypropylcellulose des Typs Klucel ® HXF des Herstellers Hercules, Polyisobutylen vom Typ Oppanol ® B 15 SF der Firma BASF AG, Dimethylisosorbid vom Typ Arlasolve ® DMI der Firma ICI Surfactionts, Polyinylpyrrolidon vom Typ Kollidon 12 PF sowie das Vinylacetat-vinylpyrrolidon-Copolymere vom Typ Kollidon VA 64 der Firma BASF AG.

### Beispiel 1

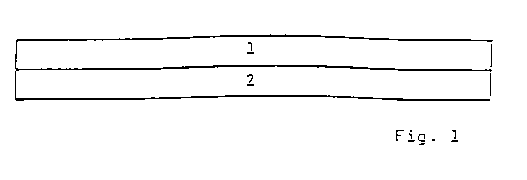

In 62,4 g einer 50 %igen Lösung von Silikonklebstoff in Benzin werden unter Rühren nacheinander
- 0,8 g: Desogestrel
- 8,0 g: Dimethylisosorbid
eingetragen. Nach Entgasen des Ansatzes wird die Mischung mittels einer Beschichtungsvorrichtung auf Polyesterfolie in der Weise aufgetragen, daß nach Entfernung des flüchtigen Lösemittels ein gleichmäßiger Film von 40 g/m² Feststoffauftrag entsteht. Anschließend wird mit einem fluorpolymerbeschichteten Polyester-Liner kaschiert. Das so erhaltene Laminat wird mittels einer Stanzvorrichtung in runde Einzelpflaster von 10 cm² Fläche geteilt und in Aluminiumfolie verpackt. Fig. 1 zeigt einen Querschnitt durch dieses Pflaster ohne Polyester-Liner. Das Pflaster haftet nach Abziehen der Liner-Folie auf der Haut.

Die Gehaltsbestimmung ergibt eine gleichmäßige Wirkstoffverteilung von 0,08 mg/cm² im Mittel.

### Beispiel 2 In 170 g einer 50 %igen Lösung von Polyacrylester-Klebstoff in Aceton/Benzin werden nacheinander

- 5,0 g: Gestoden
- 10,0 g: Dimethylisosorbid
unter Rühren gelöst. Nach Entgasen des Ansatzes wird die Lösung mittels einer Beschichtungsvorrichtung auf Polyesterfolie in der Weise aufgetragen, daß nach Entfernung der flüchtigen Lösemittel ein gleichmäßiger Film von 100 g/m² Feststoffauftrag entsteht. Anschließend wird mit einer silikonisierten wirkstofffreien Liner-Folie kaschiert. Das so erhaltene Laminat wird mittels einer Stanzvorrichtung in Einzelpflaster von 10 cm² Fläche geteilt und in Aluminiumfolie verpackt. Das Pflaster haftet nach Abziehen der Liner-Folie auf der Haut.

Der Gehalt an Gestoden beträgt im Mittel 0,5 mg/cm².

### Beispiel 3

In 112 g einer 50 %igen Lösung von Polyacrylester-Klebstoff in Aceton/Benzin werden nacheinander
- 3,5 g: Estradiol
- 3,5 g: Levonorgestrel und
- 7,0 g: Dimethylisosorbid mit 10% Laurinsäure
unter Rühren gelöst bzw. suspendiert. Nach Entgasen des Ansatzes wird die Mischung mittels einer Beschichtungsvorrichtung auf Polyesterfolie in der Weise aufgetragen, daß nach Entfernung der flüchtigen Lösemittel ein gleichmäßiger Film von 70 g/m² Feststoffauftrag entsteht Anschließend wird mit einer silikonisierten wirkstofffreien Liner-Folie kaschiert. Das so erhaltene Laminat wird mittels einer Stanzvorrichtung in Einzelpflaster von 10 cm² Fläche geteilt und in Aluminiumfolie verpackt. Das Pflaster haftet nach Abziehen der Liner-Folie auf der Haut.

Der Gehalt an Estradiol und Levonorgestrel liegt gleichermaßen bei je 0,35 mg/cm².

### Beispiel 4

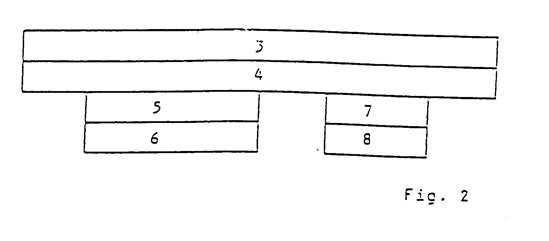

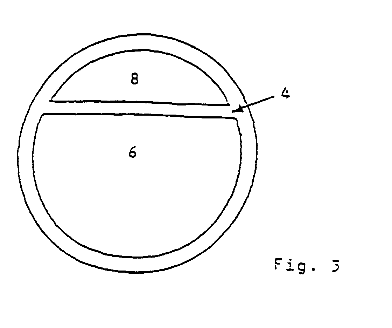

Analog Beispiel 1 werden zwei unterschiedliche segmentartige Matrixsysteme hergestellt, die die in Fig. 2 und 3 dargestellte Formgebung haben. Das Matrixsystem 1 besteht aus der mit einer Polyesterfolie 7 versehenen Matrixschicht 8 folgender Zusammensetzung
- 1,0 mg: Norethisteronacetat
- 5,0 mg: Dimethylisosorbid und
- 44 mg: Acrylatkleberfeststoff
und hat eine Fläche von 5 cm². Das Matrixsystem II besteht aus der mit einer Polyesterfolie 5 versehenen Matrixschicht 6 folgender Zusammensetzung
- 2,0 mg: Ethinylestrasdiol
- 10,0 mg: Dimethylisosorbid und
- 88 mg: Acrylatkleberfeststoffe
und hat eine Fläche von 10 cm².

Beide Matrixsysteme werden auf eine mit Hauthaftkleber beschichtete Leinwand aufgeklebt, wie Fig. 3 zeigt. Nach Kaschieren und Ausstanzen entstehen so Pflaster der in Fig. 2 und 3 gezeigten Art.

### Beispiel 5

In 100 g einer 50%igen Lösung von Polyacrylester-Klebstoff in Ethylacetat/Isopropanol werden nacheinander
- 1,0 g: Gestoden
- 8,0 g: Dimethylisosorbid und
- 9,0 g: Kollidon 12 PF
unter Rühren gelost Nach Entgasen des Ansatzes wird die Mischung mittels einer Beschichtungsvorrichtung auf Polyesterfolie in der Weise aufgetragen, daß nach Entfernen des flüchtigen Lösungsmittels ein gleichmäßiger Film von 80 g / m² Feststoffauftrag entsteht Anschließend wird mit einer silikonisierten, wirkstofffreien Liner-Folie kaschiert. Das so erhaltene Laminat wird mittels einer Stanzvorrichtung in Einzelpflaster von 15 cm² geteilt und in Aluminiumfolie verpackt. Das Pflaster haftet nach Abziehen der Linerfolie auf der Haut.

### Beispiel 6

In 100 g einer 50%igen Lösung von Polyacrylester-Klebstoff in Ethylacetat/Isopropanol werden nacheinander
- 1,0 g: Gestoden
- 2,0 g: Estradiol
- 8,0 g: Dimethylisosorbid und
- 9,0 g: Kollidon 12 PF unter Rühren gelöst Nach Entgasen des Ansatzes wird die Mischung mittels einer Beschichtungsvorrichtung auf Polyesterfolie in der Weise aufgetragen, daß nach Entfernen des flüchtigen Lösungsmittels ein gleichmäßiger Film von 80 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 15 cm² geteilt und in Aluminiumfolie verpackt. Das Pflaster haftet nach Abziehen der Linerfolie auf der Haut.

### Beispiel 7

In 100 g einer 50%igen Lösung von Polyacrylester-Klebstoff in Ethylacetat/Isopropanol werden nacheinander
- 1,0 g: Levonorgestrel
- 8,0 g: Dimethylisosorbid und
- 9,0 g: Kollidon 12 PF
unter Rühren gelöst Nach Entgasen des Ansatzes wird die Mischung mittels einer Beschichtungsvorrichtung auf Polyesterfolie in der Weise aufgetragen, daß nach Entfernen des flüchtigen Lösungsmittels ein gleichmäßiger Film von 80 g / m² Feststoffauftrag entsteht Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfolie verpackt. Das Pflaster haftet nach Abziehen der Linerfolie auf der Haut.

### Beispiel 8

In 100 g einer 50%igen Lösung von Polyacrylester-Klebstoff in Ethylacetat/Isopropanol werden nacheinander
- 1,0 g: Levonorgestrel
- 1,0 g: Estradiol
- 8,0 g: Dimethylisosorbid und
- 9,0 g: Kollidon 12 PF
unter Rühren gelöst. Nach Entgasen des Ansatzes wird die Mischung mittels einer Beschichtungsvorrichtung auf Polyesterfolie in der Weise aufgetragen, daß nach Entfernen des flüchtigen Lösungsmittels ein gleichmäßiger Film von 80 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfolie verpackt. Das Pflaster haftet nach Abziehen der Linerfolie auf der Haut.

### Beispiel 9

In 100 g einer 50%igen Lösung von Polyacrylester-Klebstoff in Ethylacetat/Isopropanol werden nacheinander
- 2,0 g: Estradiol
- 8,0 g: Dimethylisosorbid und
- 9,0 g: Kollidon VA 64
unter Rühren gelöst. Nach Entgasen des Ansatzes wird die Mischung mittels einer Beschichtungsvorrichtung auf Polyesterfolie in der Weise aufgetragen, daß nach Entfernen des flüchtigen Lösungsmittels ein gleichmäßiger Film von 80 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 15 cm² geteilt und in Aluminiumfolie verpackt. Das Pflaster haftet nach Abziehen der Linerfolie auf der Haut.

### Beispiel 10

In 170 g einer 38%igen Lösung von Polyacrylester Klebstoff in Ethylacetat/Isopropanol werden nacheinander
- 0,9 g: Levonorgestrel
- 1,8 g: Estradiol
- 19,0 g: Dimethylisosorbid
- 4,5 g: Laurinsäure und
- 13,5 g: Kollidon VA 64
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

### Beispiel 11

In 170 g einer 38%igen Lösung von Polyacrylester Klebstoff in Ethylacetat/Isopropanol werden nacheinander
- 0,9 g: Levonorgestrel
- 19,0 g: Dimethylisosorbid
- 4,5 g: Laurinsäure und
- 13,5 g: Kollidon VA 64
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

### Beispiel 12

In 170 g einer 38%igen Lösung von Polyacrylester Klebstoff in Ethylacetat/Isopropanol werden nacheinander
- 0,9 g: Gestoden
- 1,8 g: Estradiol
- 19,0 g: Dimethylisosorbid
- 4,5 g: Laurinsäure und
- 13,5 g: Kollidon VA 64
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

### Beispiel 13

In 170 g einer 38%igen Lösung von Polyacrylester Klebstoff in Ethylacetat/Isopropanol werden nacheinander
- 0,9 g: Gestoden
- 19,0 g: Dimethylisosorbid
- 4,5 g: Laurinsäure und
- 13,5 g: Kollidon VA 64
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

### Beispiel 14

In 170 g einer 38%igen Lösung von Polyacrylester Klebstoff in Ethylacetat/Isopropanol werden nacheinander
- 0,9 g: Levonorgestrel
- 1,8 g: Estradiol
- 19,0 g: Dimethylisosorbid
- 4,5 g: Laurinsäure und
- 13,5 g: Kollidon 12 PF
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

### Beispiel 15

In 170 g einer 38%igen Lösung von Polyacrylester Klebstoff in Ethylacetat/Isopropanol werden nacheinander
- 0,9 g: Levonorgestrel
- 19,0 g: Dimethylisosorbid
- 4,5 g: Laurinsäure und
- 13,5 g: Kollidon 12 PF
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

### Beispiel 16

In 170 g einer 38%igen Lösung von Polyacrylester Klebstoff in Ethylacetat/Isopropanol werden nacheinander
- 0,9 g: Gestoden
- 1,8 g: Estradiol
- 19,0 g: Dimethylisosorbid
- 4,5 g: Laurinsäure und
- 13,5 g: Kollidon 12 PF
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

### Beispiel 17

In 170 g einer 38%igen Lösung von Polyacrylester Klebstoff in Ethylacetat/Isopropanol werden nacheinander
- 0,9 g: Gestoden
- 19,0 g: Dimethylisosorbid
- 4,5 g: Laurinsäure und
- 13,5 g: Kollidon 12 PF
unter Rühren gelöst. Nach Entgasen des Ansatzes wird die Mischung mittels einer Beschichtungseinrichtung auf Polyesterfolie in der Weise aufgetragen, daß nach Entfernen des flüchtigen Lösungsmittels ein gleichmäßiger Film von 100 g / m² Feststoffauftrag entsteht. Anschließend wird mit einer silikonisierten, wirkstofffreien Liner-Folie kaschiert. Das so erhaltene Laminat wird mittels einer Stanzvorrichtung in Einzelpflaster von 20 cm² geteilt und in Aluminiumfoie verpackt. Das Pflaster haftet nach Abziehen der Linerfolie auf der Haut.

## Patentansprüche

1. Transdermale therapeutische Systeme, enthaltend Sexualsteroide und gegebenenfalls Penetrationsverstärker und Kristallisationsinhibitoren, dadurch gekennzeichnet, daß sie 1 bis 40 Gewichtsprozent Dimethylisosorbid bezogen auf die gesamte Wirkstoffphase enthalten, mit Ausnahme von Systemen, die wirkstoffhaltige, nicht fließfähige Gelphasen oder 3 Keto-desogestrel enthalten.

2. Transdermale therapeutische Systeme gemäß Patentanspruch 1, dadurch gekennzeichnet, daß sie 5 bis 25 Gewichtsprozent Dimethylisosorbid bezogen auf die gesamte Wirkstoffphase enthalten.

3. Transdermale therapeutische Systeme gemäß einem der Patentansprüche 1 oder 2, dadurch gekennzeichnet, daß sie als Sexualsteroid ein Gestagen und/oder ein Östrogen enthalten.

4. Transdermale therapeutische Systeme gemäß Patentanspruch 3, dadurch gekennzeichnet, daß sie als Gestagen Gestoden, Levonorgestrel, Desogestrel, Norethisteron oder Norethisteronacetat enthalten.

5. Transdermale therapeutische Systeme gemäß Patentanspruch 3, dadurch gekennzeichnet, daß sie als Östrogen(e) Estradiol, Estriol, 17 alpha-Ethinyl-estradiol, Mestranol, 14 alpha, 17 alpha-Ethanoestra-1,3,5(10)-trien-3, 17 beta-diol, 14 alpha, 17 alpha-Ethanoestra-1,3,5(10)-trien-3, 16 alpha, 17 beta-triol oder Ester dieser Verbindungen enthalten.

6. Transdermale therapeutische Systeme gemäß einem der Patentansprüche 1 - 5 , dadurch gekennzeichnet, daß sie aus
(a) einer undurchlässigen Deckschicht,
ein bis drei an der Deckschicht haftenden, das Gestagen und/oder Östrogen und das Dimethylisosorbid und gewünschtenfalls penetrationsverstärkende Mittel und Kristallisationsinhibitoren einem gewünschtenfalls penetrationsverstärkende Mittel enthaltenden Hauthaftkleber abgedeckten oder umgebenen Matrixschicht(en), einer abziehbaren Schutzschicht, oder
(b) einer mit einem gewünschtenfalls penetrationsverstärkende Mittel enthaltenden Haftkleber versehenen Abdeckung, ein oder zwei eine Haftkleberumrandung unbedeckt lassende, mittels einer undurchlässigen Abdeckung an dem Haftkleber befestigten, das Gestagen und/oder Östrogen und das Dimethylisosorbid und gewünschtenfalls penetrationsverstärkende Mittel und Kristallisationsinhibitoren enthaltende Matrixschicht(en) und einer abziehbaren Schutzschicht, besteht.

7. Mittel zur transdermalen Applikation gemäß Patentanspruch 6, dadurch gekennzeichnet, daß es eine wirkstoffhaltige Matrixschicht enthält.

8. Mittel zur transdermalen Applikation gemäß Patentanspruch 7, dadurch gekennzeichnet, daß es zwei oder drei wirkstoffhaltige Matrixschichten enthält.

9. Mittel zur transdermalen Applikation gemäß Patentanspruch 8, dadurch gekennzeichnet, daß die wirkstoffhaltigen Matrixschichten unterschiedliche Wirkstoffe enthalten.

10. Verwendung von östrogenfreien Mitteln zur transdermalen Applikation gemäß einem der Patentansprüche 1 bis 9 zur Herstellung eines Arzneimittels zur transdermalen Kontrazeption, zur Behandlung der Endometriose, zur Behandlung von gestagenabhängigen Tumoren und zur Behandlung des prämenstruellen Syndroms.

11. Verwendung von Mitteln zur transdermalen Applikation gemäß einem der Patentansprüche 1 bis 9 gegebenenfalls in Kombination mit östrogenhaltigen Mitteln zur Herstellung eines Arzneimittels zur Behandlung klimakterischer Beschwerden, zur Prävention der Osteoporose, zur Zyklusregulierung, zur Zyklusstabilisierung und zur transdermalen Kontrazeption.

## Claims

1. Transdermal therapeutic systems, comprising sex steroids and, if appropriate, penetration enhancers and crystallization inhibitors, characterized in that they contain 1 to 40% by weight of dimethylisosorbide based on the total active compound phase, with the exception of systems which contain active compound-containing, non-fluid gel phases or 3-ketodesogestrel.

2. Transdermal therapeutic systems according to Patent Claim 1, characterized in that they contain 5 to 25% by weight of dimethylisosorbide based on the total active compound phase.

3. Transdermal therapeutic systems according to one of Patent Claims 1 or 2, characterized in that they contain a gestagen and/or an oestrogen as sex steroid.

4. Transdermal therapeutic systems according to Patent Claim 3, characterized in that they contain gestodene, levonorgestrel, desogestrel, norethisterone or norethisterone acetate as gestagen.

5. Transdermal therapeutic systems according to Patent Claim 3, characterized in that they contain oestradiol, oestriol, 17α-ethinyloestradiol, mestranol, 14α,17α-ethanoestra-1,3,5(10)-triene-3,17β-diol, 14α,17α-ethanoestra-1,3,5(10)-triene-3,16α,17β-triol or esters of these compounds as oestrogen(s).

6. Transdermal therapeutic systems according to one of Patent Claims 1-5, characterized in that they consist of
(a) an impermeable covering layer,
one to three matrix layers adhering to the covering layer, containing the gestagen and/or oestrogen and the dimethylisosorbide and, if desired, penetration-enhancing agents and crystallization inhibitors, which are permeable for these components and self-adhering or covered or surrounded by a skin adhesive which, if desired, contains penetration-enhancing agents, a removable protective layer, or
(b) a covering provided with an adhesive which, if desired, contains penetration-enhancing agents, one or two matrix layers leaving an adhesive border uncovered, attached to the adhesive by means of an impermeable covering, containing the gestagen and/or oestrogen and the dimethylisosorbide and, if desired, penetration-enhancing agents and crystallization inhibitors, and a removable protective layer.

7. Agent for transdermal application according to Patent Claim 6, characterized in that it contains an active compound-containing matrix layer.

8. Agent for transdermal application according to Patent Claim 7, characterized in that it contains 2 or 3 active compound-containing matrix layers.

9. Agent for transdermal application according to Patent Claim 8, characterized in that the active compound-containing matrix layers contain different active compounds.

10. Use of oestrogen-free agents for transdermal application according to one of Patent Claims 1 to 9 for the production of a medicament for transdermal contraception, for the treatment of endometriosis, for the treatment of gestagen-dependent tumours and for the treatment of premenstrual syndrome.

11. Use of agents for transdermal application according to one of Patent Claims 1 to 9, if appropriate in combination with oestrogen-containing agents, for the production of a medicament for the treatment of climacteric symptoms, for the prevention of osteoporosis, for cycle regulation, for cycle stabilization and for transdermal contraception.

## Revendications

1. Systèmes thérapeutiques transdermiques contenant des stéroïdes sexuels et éventuellement des agents favorisant la pénétration et des inhibiteurs de cristallisation, caractérisés en ce qu'ils contiennent de 1 à 40 pour cent en poids de diméthylisosorbide, par rapport à la totalité de la phase de la substance active, à l'exception de systèmes qui contiennent des phases de gel non fluides renfermant une substance active, ou du 3-cétodésogestrel.

2. Systèmes thérapeutiques transdermiques selon la revendication 1, caractérisés en ce qu'ils contiennent de 5 à 25 pour cent en poids de diméthylisosorbide, par rapport à la totalité de la phase de la substance active.

3. Systèmes thérapeutiques transdermiques selon l'une quelconque des revendications 1 ou 2, caractérisés en ce qu'ils contiennent un gestagène et/ou un oestrogène en tant que stéroïde sexuel.

4. Systèmes thérapeutiques transdermiques selon la revendication 3, caractérisés en ce qu'ils contiennent du gestodène, du lévonorgestrel, du désogestrel, de la noréthistérone ou de l'acétate de noréthistérone en tant que gestagène.

5. Systèmes thérapeutiques transdermiques selon la revendication 3, caractérisés en ce qu'ils contiennent de l'oestradiol, de l'oestriol, du 17α-éthinyl-oestradiol, du mestranol, du 14α, 17α-éthanoestra-1,3,5(10)-triène-3,17β-diol, du 14α, 17α-éthanoestra-1,3,5(10)-triène-3,16 α, 17β-triol ou des esters de ces composés en tant qu'oestrogène(s).

6. Systèmes thérapeutiques transdermiques selon l'une quelconque des revendications 1 à 5, caractérisés en ce qu'ils sont constitués de
(a) une couche recouvrante imperméable,
une à trois couche(s) matricielle(s) adhérant à la couche recouvrante, contenant le gestagène et/ou l'oestrogène et le diméthylisosorbide et éventuellement des agents favorisant la pénétration et des inhibiteurs de cristallisation, et recouverte(s) ou entourée(s) d'un adhésif pour la peau contenant éventuellement des agents favorisant la pénétration, une couche protectrice pelable, ou
(b) un recouvrement pourvu d'un adhésif pour la peau contenant éventuellement des agents favorisant la pénétration, une ou deux couche(s) matricielle(s) laissant un bord de l'adhésif non recouvert fixée(s) à l'adhésif au moyen d'un recouvrement imperméable, et contenant le gestagène et/ou l'oestrogène et le diméthylisosorbide et éventuellement des agents favorisant la pénétration et des inhibiteurs de cristallisation et une couche protectrice pelable.

7. Produit pour application transdermique selon la revendication 6, caractérisé en ce qu'il contient une couche matricielle renfermant une substance active.

8. Produit pour application transdermique selon la revendication 7, caractérisé en ce qu'il contient deux ou trois couches matricielles renfermant une substance active.

9. produit pour application transdermique selon la revendication 8, caractérisé en ce que les couches matricielles renfermant une substance active contiennent des substances actives différentes.

10. Utilisation de produits sans oestrogène pour application transdermique selon l'une quelconque des revendications 1 à 9 pour la préparation d'un médicament destiné à la contraception transdermique, au traitement de l'endométriose, au traitement de tumeurs liées aux gestagènes et au traitement du syndrome prémenstruel.

11. Utilisation de produits pour application transdermique selon l'une quelconque des revendications 1 à 9, éventuellement en combinaison avec des produits contenant des oestrogènes, pour la préparation d'un médicament destiné au traitement de symptômes climatériques, à la prévention de l'ostéoporose, à la régulation du cycle, à la stabilisation du cycle et à la contraception transdermique.
